# EUROPÄISCHE PATENTSCHRIFT

(11) **EP 2 583 620 B1**
(45) Veröffentlichungstag und Bekanntmachung des Hinweises auf die Patenterteilung: **23.08.2017**
(21) Anmeldenummer: 13405008.7
(22) Anmeldetag: 24.01.2013
(51) Int. Cl.: A61B 3/107

(54) **Topograph**
Topograph
Topographe

(43) Veröffentlichungstag der Anmeldung: 24.04.2013
(73) Patentinhaber: HAAG-STREIT AG, CH-3098 Köniz (CH)
(72) Erfinder: Breitenstein, Jörg, 3052 Zollikofen (CH); Haehnle, Jonas, 3011 Bern (CH); Zoss, Christian, 3123 Belp (CH); Stalder, Peter, 4805 Brittnau (CH); Baltzer, Kaspar, 3014 Bern (CH); Rindlisbacher, Ernst, 3067 Boll (CH); Meznaric, André, 3008 Bern (CH); Schläppi, Christian, 3018 Bern (CH); Robledo, Lucio, 3013 Bern (CH)
(74) Vertreter: Stäbler, Roman

(56) Entgegenhaltungen:
- US-A1- 2006 147 189
- US-A1- 2010 118 270

## Beschreibung

### Technisches Gebiet

Die Erfindung betrifft einen Adapter für eine Vorrichtung zum Messen einer ersten Eigenschaft eines Auges. Weiter betrifft die Erfindung eine Anordnung zum Messen von Eigenschaften eines Auges, umfassend einen Adapter und eine Vorrichtung zum Messen einer ersten Eigenschaft des Auges. Schliesslich betrifft die Erfindung ein Verfahren zum Betreiben der Anordnung.

### Stand der Technik

Für die optimale Anpassung von Intraokularlinsen (IOLs) ist es nötig, vor der Operation die geometrischen Eigenschaften des zu operierenden Auges mit hoher Genauigkeit zu bestimmen. Gängige Formeln zur Anpassung von IOLs basieren einerseits auf axialen Abständen des Auges, wie zum Beispiel Augenlänge und Vorderkammertiefe, sowie andererseits Krümmungsradien der Grenzflächen, insbesondere Krümmungsradius und Achse des flachsten und des steilsten Meridians der Hornhautvorderseite.

Im vorderen Augenabschnitt werden zur Abstandsmessung heute zum Beispiel interferometrische oder schnittbildgenerierende Verfahren eingesetzt, insbesondere eine spaltförmige Beleuchtung der Hornhaut in Verbindung mit der Abbildung eines Schnittbilds auf einer Kamera, deren Sensor und Linse in Scheimpfluggeometrie angeordnet sind.

Zur Bestimmung der Krümmungsradien des Auges werden meist Reflektometrieverfahren eingesetzt, welche auf der Abbildung der durch die Hornhautvorderseite (oder anderer Strukturen des Auges) hervorgerufenen Spiegelreflexe eines bekannten Lichtmusters auf einen digitalen Kamerasensor basieren.

In einer weiteren Methode wird eine spaltförmige Beleuchtung lateral über das Auge geführt; die Topographie der beleuchteten Augenstrukturen kann dann wiederum durch eine Analyse der Reflektionsbilder bestimmt werden.

Es ist prinzipiell möglich, Messungen dieser komplementären Daten (axiale Abstände und Krümmungen) im selben Gerät zu kombinieren. So sind Geräte wie der Lenstar LS900 (Haag-Streit) oder IOL Master (Zeiss) in der Lage, axiale Längenmessungen im Auge sowie Keratometriemessungen (Krümmung und Achse des steilen und flachen Meridians) durchzuführen. Weiterhin ist in der Patentschrift DE 19852331 (A1) die Kombination aus Placidoring-Topographiemessung und optischer Zeitdomänen-Kohärenztomographie bekannt.

Allerdings haben diese Geräte den Nachteil, dass sie auf eine spezifische Implementierung der einzelnen Gerättypen eingeschränkt sind.

Ein solches Gerät zur exakten Bestimmung von Augenlängen und Topographien benötigt zudem entweder einen wesentlich kürzeren Arbeitsabstand (was bei unruhigen Patienten die Messung erschwert und das Risiko einer Kollision des Auges mit dem Messgerät erhöht) oder deutlich grösseren Gerätedurchmesser, was den Sichtkontakt zwischen Bedienperson und Patient erschwert und zu einem erhöhten Kollisionsrisiko zwischen Messgerät und Stirn oder Nasenbereich des Patienten führt.

Um die zur IOL-Anpassung benötigten Parameter wie axiale Augenlänge und Hornhautkrümmungen zu bestimmen, werden nach dem Stand der Technik also entweder mindestens zwei spezialisierte Messgeräte, unflexible Kombinationsgeräte oder kostspielige und komplexe 3D-Tomographen benötigt. Diese Lösungen sind sowohl unter ergonomischen als auch ökonomischen Gesichtspunkten nicht zufriedenstellend. Weiterhin ist es nach Stand der Technik nicht möglich, die Messmethode flexibel an die Patienten anzupassen und einen individuellen Kompromiss für Arbeitsabstand, minimale Blendung des Patienten, Informationsgehalt und Genauigkeit der Messung zu wählen. US 2010/118270 und US 2006/147189 offenbaren jeweils eine Funduskamera mit einem abnehmbaren Adapter zur Messung von Eigenschaften der Vorderkammer bzw. der Pupillengröße.

### Darstellung der Erfindung

Aufgabe der Erfindung ist es, eine dem eingangs genannten technischen Gebiet zugehörende Anordnung zum Messen von Eigenschaften eines Auges zu schaffen, welche besonders einfach aufgebaut, variabel einsetzbar und kostengünstig ist.

Die Lösung der Aufgabe ist durch die Merkmale des Anspruchs 1 definiert. Gemäss der Erfindung ist der Adapter, vorzugsweise lösbar, an der Vorrichtung befestigbar, wobei bei, an der Vorrichtung befestigtem Adapter, eine zweite zusätzliche Eigenschaft des Auges messbar ist.

Eine Anordnung zum Messen von Eigenschaften eines Auges umfasst einen Adapter und eine Vorrichtung zum Messen einer ersten Eigenschaft des Auges, wobei der Adapter, vorzugsweise lösbar, an der Vorrichtung befestigbar ist und wobei bei, an der Vorrichtung befestigtem Adapter, eine zweite Eigenschaft des Auges messbar ist.

Unter dem Begriff Adapter wird nachfolgend ein Gerät, einen Aufsatz oder dergleichen verstanden, mit welchem in Bezug auf die Vorrichtung eine Eigenschaft gemessen werden kann, welche mit der Vorrichtung alleine, also ohne den Adapter, nicht oder nicht in derselben Qualität gemessen werden kann. Vorzugsweise sind die Eigenschaften, welche durch die Vorrichtung gemessen werden können, aber unterschiedlich zu denjenigen Eigenschaften, welche mit der Vorrichtung unter Verwendung des Adapters gemessen werden können.

Der Adapter ist vorzugsweise derart ausgebildet, dass er Ressourcen der Vorrichtung verwenden kann. Damit können gewisse Bauteile, welche für die Messung der ersten wie auch der zweiten Eigenschaft notwendig sind, für beide Messungen verwendet werden, so dass das Gerät besonders kostengünstig hergestellt werden kann.

Die Möglichkeit dieselbe Vorrichtung wahlweise mit dem Adapter für verschiedene Messmethoden zu nutzen, reduziert dabei auch das Risiko systematischer Messfehler. Weiter wird damit das Referenzieren der Messungen anhand (nahezu) zeitgleich erfasster gemeinsamer Daten ermöglicht und somit auch die Vergleichbarkeit der Daten verbessert. Insbesondere besteht damit die Möglichkeit, die ermittelten Daten der ersten und zweiten Eigenschaft kombiniert zu analysieren und so effizient umfassendere Aussagen über das Messobjekt machen zu können. Weiterhin wird somit die Gesamtdauer der Messungen zum Vorteil von Patienten und medizinischem Personal auf ein Minimum reduziert.

Darüber hinaus erfordert die Anordnung keine zusätzliche Standfläche, sondern lediglich einen Aufbewahrungsort für den Adapter. Der klinische Arbeitsplatz kann daher optimal genutzt werden. Weiter wird bei der Messung der ersten Eigenschaft der visuelle Kontakt zum Patienten durch den Adapter nicht zwingend eingeschränkt.

Der Adapter kann als Zusatzgerät für bestehende Vorrichtungen zum Messen der ersten Eigenschaft eines Auges vorgesehen sein. Weiter können für die Vorrichtung auch mehrere unterschiedliche Adapter vorgesehen sein, welche Messungen unterschiedlicher Eigenschaften eines Auges ermöglichen. Weiter kann durch den modularen Aufbau einem Kunden beim Erwerb der Vorrichtung die Option geboten werden, sich zu einem späteren Zeitpunkt noch für den Adapter für die Messung der zweiten Eigenschaft des Auges zu entscheiden.

Die Anordnung, umfassend die Vorrichtung und den Adapter, ist weiter vorteilhaft, wenn für die Messung der beiden Eigenschaften unterschiedliche Arbeitsabstände notwendig sind. So können zum Beispiel der Adapter und die Vorrichtung derart ausgebildet sein, dass für die zweite Eigenschaft ein kleinerer Abstand zum Messobjekt erforderlich ist, wobei der kleinere Abstand durch das Positionieren des Adapters erreicht werden kann.

Dass bei an der Vorrichtung befestigtem Adapter die zweite Eigenschaft des Auges messbar ist, ist nicht zwangsläufig dahingehend auszulegen, dass die erste Eigenschaft nur dann messbar ist, wenn der Adapter nicht an der Vorrichtung befestigt ist. Es ist denkbar, dass der an der Vorrichtung befestigte Adapter zwischen einem aktiven und einem passiven Zustand schaltbar ist. In einer bevorzugten Ausführungsform ist allerdings der Adapter als abnehmbarer Aufsatz für die Vorrichtung ausgebildet. In einer bevorzugten Ausführungsform können bei befestigtem Adapter die erste und die zweite Eigenschaft, insbesondere simultan respektive gleichzeitig, gemessen werden. Typischerweise wird der Adapter für die Messung zwischen dem Patienten und der Vorrichtung an letzterer befestigt.

Dem Fachmann ist klar, dass statt des Auges auch ein Modell eines Auges, zum Beispiel ein Glasauge, ein Linsensystem oder allgemein ein zumindest teilweise, bei mindestens einer Wellenlänge, lichtdurchlässiger Körper mit der Vorrichtung und dem Adapter auf die beiden Eigenschaften untersucht werden kann.

Bei der Eigenschaft handelt es sich vorzugsweise um eine optische messbare Eigenschaft, wobei mit optisch nicht ausschliesslich der visuelle Anteil des optischen Spektrums gemeint ist. Die optische Eigenschaft kann auch ein Spektrum im infraroten oder ultravioletten Spektralbereich umfassen. In Varianten können aber auch andere Eigenschaften, welche zum Beispiel durch akustische Messmethoden wie Ultraschall messbar sind, gemessen werden. Vorzugsweise handelt es sich bei den optischen Eigenschaften um Längen, Krümmungen, Topographien etc.

Unter dem Begriff Messen wird jegliches Bestimmen eines Parameters verstanden, insbesondere kann darunter eine quantitative im Wesentlichen stufenlose Messung oder eine diskrete Messung, aber auch eine binäre Messung wie erfüllt/nicht erfüllt etc. verstanden werden.

Erfindungsgemäss ist die erste und/oder die zweite Eigenschaft eine geometrische Eigenschaft des Auges. Damit können mit einer Vorrichtung, unter Verwendung des Adapters unterschiedliche Messungen durchgeführt werden, worauf mit den Messdaten zum Beispiel ein Auge charakterisiert werden kann. Zum Beispiel kann damit ein geometrisches Modell eines Auges erstellt werden.

In Varianten können die erste und/oder die zweite Eigenschaft auch zum Beispiel ein Brechungsindex, ein Farbspektrum, einen ortsabhängigen Brechungsindex, Streu-, Reflektions- oder Absorptionskoeffizienten, ein Farbspektrum, auch jeweils als Funktion der Polarisation und dergleichen umfassen.

Erfindungsgemäss umfasst die erste Eigenschaft einen Abstande in einem Auge und die zweite Eigenschaft zumindest eine Topographie oder eine Krümmung des Auges. Damit kann ein dreidimensionales Modell des vermessenen Auges erstellt werden. Das Modell kann zum Beispiel bei der Anpassung von Intraokularlinsen oder zur Unterstützung von Operationen am Auge eingesetzt werden. Weiter können mit solchen Modellen auch Strahlengänge durch das Auge simuliert werden. Prinzipiell können auch weitere Eigenschaften durch die Vorrichtung oder den Adapter erfasst werden, insbesondere ist dem Fachmann klar, dass auch abgeleitete Grössen aus diesen Daten ermittelt werden können, wie zum Beispiel ein Volumen und dergleichen.

In Varianten kann auch die erste Eigenschaft eine Topographie umfassen und die zweite Eigenschaft einen Abstand im Auge umfassen.

Der Adapter kann zum Beispiel als ein Reflektometriemodul ausgebildet sein, welches eine Struktur umfasst, die ein Lichtmuster formt, um anhand der Reflektion dieses Musters durch das Auge Rückschlüsse auf deren geometrische Eigenschaften zu ziehen. Reflektometriemodul ist in diesem Fall als vorzugsweise abnehmbarer Adapter zur Verwendung an einer Vorrichtung, respektive einem Gerät zur axialen Abstandsmessung im Auge gestaltet, wobei das Reflektometriemodul nicht zwangsläufig alle notwendigen Komponenten umfassen muss, um einen autonomen Betrieb zu ermöglichen.

Bevorzugt umfasst die erste Eigenschaft mindestens eine der nachfolgenden Längen: Hornhautdicke, Vorderkammertiefe, Linsendicke, Augenlänge. Die erste Eigenschaft kann natürlich auch weitere Daten, insbesondere Längen umfassen, wie zum Beispiel die Tiefe der hinteren Augenkammer etc. Dem Fachmann ist klar, dass bei einer Messung unterschiedlicher Längen auch weitere direkt abgeleitet werden können, so dass diese auch indirekt bestimmt werden können. Ebenso kann unter Verwendung von bekannten Daten, zum Beispiel empirisch ermittelten Längenverhältnissen, auch auf weitere Längen geschlossen werden.

In Varianten kann die erste Eigenschaft auch statt der Längen andere Eigenschaften umfassen, wie zum Beispiel Topographiedaten.

Vorzugsweise umfasst der Adapter Kopplungsmittel, womit der Adapter an der Vorrichtung befestigbar ist, wobei die Kopplungsmittel vorzugsweise einen Magneten umfassen. Mit den Kopplungsmitteln wird vorzugsweise eine einfache Montage und Demontage des Adapters an der Vorrichtung ermöglicht. Damit wird erreicht, dass effizient zwischen den beiden Messmodi, nämlich zwischen der Messung der ersten Eigenschaft, oder weiteren Eigenschaften, die von der Vorrichtung erfasst werden können, und der zweiten Eigenschaft gewechselt werden kann. So kann zum Beispiel bei abgenommenem Adapter eine keratometrische Messung und bei aufgesetztem Adapter eine Topographiemessung durchgeführt werden, während eine Längenmessung in beiden Fällen (bei aufgesetztem wie auch abgenommenem Adapter) möglich ist.

Besonders bevorzugt umfassen dazu die Kopplungsmittel einen Magneten, womit die Befestigung und das Entfernen des Adapters besonders effizient erfolgen können. Bevorzugt umfasst dazu die Vorrichtung einen Magneten. Der Adapter kann zusätzlich über einen Formschluss an der Vorrichtung in Position gehalten sein. Der Formschluss ist vorzugsweise derart gestaltet, dass der Adapter in genau einer Orientierung an der Vorrichtung in funktionsfähiger Weise befestigt werden kann. Dies kann auf verschiedenste Arten erreicht werden, zum Beispiel kann der Adapter zwei Zapfen unterschiedlichen Durchmessers aufweisen und die Vorrichtung entsprechende Löcher zur Aufnahme der Zapfen. Der Formschluss kann auch als eine Feder-Nut-Verbindung ausgebildet sein. Die Feder-Nut-Verbindung kann auch bogenförmig ausgebildet sein. Dank dem Einsatz eines Magneten kann der Formschluss als Positionierungshilfe und nur teilweise als Verbindungsvorrichtung ausgebildet sein. Weiterhin ist es zweckmässig die mechanischen Haltekräfte so zu dimensionieren, dass der Adapter nur in vollständig aufgesetztem Zustand an die Vorrichtung gekoppelt ist, beispielsweise durch geeignete Wahl der Zusammensetzung, Grösse und der Position der zur Kopplung dienenden Permanentmagneten.

Dem Fachmann ist aber klar, dass der Adapter auch über einen Clipverschluss, Bajonettverschluss und weiteren dem Fachmann bekannten reversiblen Verbindungsarten an der Vorrichtung befestigt werden kann, wobei insbesondere auf den Magneten auch verzichtet werden kann.

Das Befestigen des Adapters muss aber nicht zwingend effizient ausführbar sein, sondern kann unter Umständen durchaus auch etwas aufwendiger sein. In diesem Fall kann eine Einrichtung vorgesehen sein, womit der Adapter zwischen einer Messposition und einer passiven Position, in welcher die erste Eigenschaft gemessen werden kann, positioniert werden kann (siehe dazu weiter unten).

In Varianten kann auch lediglich die Vorrichtung Kopplungsmittel zur Befestigung des Adapters umfassen. Weiter können auch der Adapter oder der Adapter und die Vorrichtung mit einem Magneten versehen sein - in diesem Fall kann aber der Permanentmagnet des Adapters bei der Handhabung störend sein, da er magnetische Materialien anziehen würde.

Vorzugsweise umfasst der Adapter einen ersten Lichtleiter zur strukturierten Beleuchtung des Auges. Anhand der durch die strukturierte Beleuchtung erzielten Reflexionen können Rückschlüsse auf die zweiten Eigenschaften des Auges erreicht werden. Insbesondere können damit geometrische Eigenschaften, wie zum Beispiel eine Topographie des Auges, insbesondere der Hornhaut, erreicht werden. Dazu kann der Adapter über den Lichtleiter zum Beispiel mehrere regelmässig oder unregelmässig angeordnete Punkte auf das Auge projizieren. Weiter können statt der Punkte auch Ringe respektive Placidoringe projiziert werden. Anhand der von der Hornhaut reflektierten Muster kann die Topographie der Hornhaut bestimmt werden. Dem Fachmann sind auch weitere Muster bekannt, womit derselbe Zweck, nämlich eine Vermessung der Hornhauttopographie, erreicht werden kann. Dieses Messprinzip wird häufig auch Reflektometriemessung genannt - darunter fallen ebenfalls die Videokeratometrie, welche Punktprojektion verwendet, sowie die Placidoring-Topographie, welche eben konzentrische Ringe projiziert.

Der Vorteil der flächigen Beleuchtung mit der Placidoringmethode liegt in der hohen Abtastdichte und der Fähigkeit komplette Topographien zu erfassen. Weiter liefert die Placidoring-Topographie ein sehr detailliertes Modell der Hornhautoberfläche. Auch wird damit eine lokale Auswertung der Krümmungsradien an jedem Punkt (mit Ausnahme des zentralen Bereichs) ermöglicht, so dass die Hornhautvorderseite weitgehend rekonstruiert werden kann. Anhand der gemessenen Daten wird zum Beispiel die Diagnose weiterer Pathologien wie irregulären Astigmatismus ermöglicht.

Bei punktförmiger Beleuchtung lassen sich leichter die Reflektionen von tiefer liegenden Augenstrukturen (Purkinje Reflektionen) detektieren und somit auch Krümmungsradien beispielsweise der Hornhautrückseite oder der Augenlinsenvorder- und Rückseite bestimmen. Keratometriemessungen mit punktförmiger Beleuchtung liefern ein gegenüber lateralen Positionierfehlern sehr robustes Ergebnis mit exzellenter Wiederholbarkeit des Messergebnisses, sie liefern ein leicht quantifizierbares und daher vergleichbares Resultat, und haben sich somit zu Standardverfahren in der Vorbereitung zu Kataraktoperationen entwickelt.

Somit ist je nach Anwendung die Placidoringmethode oder die Punktmethode zu bevorzugen.

Alternativ lassen sich Krümmungsradien auch durch Verfahren zur Kohärenzreflektometrie gewinnen, wenn der zur Messung eingesetzte Lichtstrahl kontrolliert lateral abgelenkt wird und die daraus gewonnenen Daten zu Volumenbildern verbunden werden. Geräte nach diesem Verfahren sind jedoch sehr komplex und teuer. Schliesslich lassen sich Krümmungsradien des Auges auch anhand von Schnittbildern bestimmen. In einer weiteren Methode wird eine spaltförmige Beleuchtung lateral über das Auge geführt; die Topographie der beleuchteten Augenstrukturen kann dann wiederum durch eine Analyse der Reflektionsbilder bestimmt werden.

Vorzugsweise umfasst der Adapter einen zweiten Lichtleiter zur diffusen oder indirekten Beleuchtung des Auges. Die Beleuchtungsrichtung ist dazu vorzugsweise derart gewählt, dass ein radialer Abstand zur Messachse hinreichend gross gewählt ist, dass die direkte Reflektion durch die Hornhaut die Messung der Eigenschaften des Auges nicht beeinflussen, aber dennoch das Auge optimal beleuchtet wird. Die Lichtquelle muss nicht zwingend durch den Adapter umfasst sein, es kann auch eine Lichtquelle der Vorrichtung unter Verwendung des zweiten Lichtleiter verwendet werden.

Der Adapter kann auch weitere Lichtleiter umfassen, womit das Auge zum Beispiel von mehreren Seiten simultan diffus oder indirekt beleuchtet werden kann.

Vorzugsweise umfasst der Adapter einen zweiten Lichtleiter, womit Licht von einer Lichtquelle der Vorrichtung oder des Adapters zur diffusen und/oder indirekten Beleuchtung auf das Auge führbar ist.

In Varianten kann eine Beleuchtungseinrichtung zum diffusen oder indirekten Beleuchten auch durch den Adapter umfasst sein. In diesem Fall kann auf den zweiten Lichtleiter auch verzichtet werden.

Bevorzugt umfasst lediglich die Vorrichtung eine Lichtquelle in Form von einer oder mehrerer LED's, da diese kostengünstig und kompakt sind sowie für weitgehend beliebige Spektren zur Verfügung stehen. In Varianten können auch Laserdioden, die Licht im sichtbaren oder infraroten Spektralbereich emittieren, eingesetzt werden. Dem Fachmann sind auch weitere geeignete Lichtquellen bekannt.

Vorzugsweise umfasst die Vorrichtung eine Lichtquelle und der Adapter einen ersten Lichtleiter, so dass bei an der Vorrichtung befestigtem Adapter Licht von der Lichtquelle über den ersten Lichtleiter zum Auge leitbar ist. Die Lichtquelle der Vorrichtung kann damit sowohl für die Messung der ersten als auch der zweiten Eigenschaft des Auges verwendet werden, bzw.diesen assistieren. Dabei können die LED's zum Beispiel als Positionierhilfe, zur Dokumentation und/oder für die Keratometrie-/Topographiemessung eingesetzt werden. Damit können weiter Ressourcen eingespart werden, womit ein einfacher und kostengünstiger Aufbau des Adapters erreicht werden kann.

In Varianten kann die Lichtquelle auch durch den Adapter umfasst sein. In diesem Fall kann unter Umständen auf den ersten Lichtleiter verzichtet werden.

Bevorzugt umfasst die Vorrichtung einen Detektor, vorzugsweise eine Bildaufnahmeeinheit, womit bei an der Vorrichtung befestigtem Adapter eine zweite Eigenschaft des Auges messbar ist. Damit kann ein einziger Detektor der Vorrichtung für eine Datenerfassung verwendet werden. Der Adapter, respektive die Anordnung wird dadurch kostengünstig im Aufbau, da derselbe Detektor sowohl für die Messung der ersten als auch der zweiten Eigenschaft verwendet werden kann. Bei der Bildaufnahmeeinheit handelt es sich vorzugsweise um ein digitales Kamerasystem.

In Varianten kann statt der Vorrichtung auch der Adapter einen Detektor umfassen. Die gemeinsam verwendeten Bauteile können zum Beispiel auch lediglich durch den eingesetzten Prozessor zur Datenverarbeitung oder eine Datenausgabeeinheit wie einen Drucker, einen Bildschirm oder dergleichen gegeben sein.

Vorzugsweise umfasst die Vorrichtung einen Sensor zum Erkennen des Adapters. Der Sensor kann zum Beispiel als Signalgeber ausgebildet sein, welche an eine Datenverarbeitungseinrichtung ein Signal sendet, sobald der Adapter an der Vorrichtung als "befestigt" erkannt wird und/oder wenn der bereits befestigte Adapter in eine Betriebsstellung gebracht wird, in welcher die zweite Eigenschaft des Auges gemessen werden kann. Ebenso kann mit dem Sensor vorzugsweise erkannt werden, wenn der Adapter nicht mehr an der Vorrichtung befestigt ist respektive nicht mehr in der Betriebsstellung zum Messen der zweiten Eigenschaft des Auges ist, worauf ebenfalls ein Signal ausgegeben werden kann. Damit kann zum Beispiel ein Betriebsmodus der Andordung zwischen Messen der ersten Eigenschaft und Messen der zweiten Eigenschaft umgeschaltet werden.

Bevorzugt umfasst die Vorrichtung eine Steuereinheit, womit in Abhängigkeit eines Messwertes des Sensors ein Messmodus für die erste Eigenschaft des Auges respektive ein Messmodus für die zweite Eigenschaft des Auges schaltbar ist. Damit kann automatisch zwischen den beiden Messmodi gewechselt werden. Der Sensor kann dabei die Anwesenheit des Adapters überwachen, respektive prüfen, ob der Adapter montiert ist. Anderseits kann auch lediglich geprüft werden, ob der an der Vorrichtung befestigte Adapter in einer Position ist, in welcher die zweite Eigenschaft gemessen werden kann. Der Sensor kann als mechanischer, induktiver, elektrischer Kontaktsensor und dergleichen ausgebildet sein.

Alternativ kann auf die automatische Umschaltung des Messmodus verzichtet werden, zum Beispiel wenn in derselben Position des Adapters in der Anordnung sowohl die erste als auch die zweite Eigenschaft des Auges oder gar beide simultan gemessen werden können.

Vorzugsweise umfasst die Vorrichtung ein Interferometer, vorzugsweise ein Michelson-Interferometer. Die Verwendung eines Interferometers ermöglicht in einfacher Weise Längenmessungen im Auge vorzunehmen. Die optische Interferometrie für die axiale Abstandsmessung im Auge hat weiter den Vorteil, dass sie eine hohe Messgenauigkeit aufweist. Weiter ist das Messprinzip berührungsfrei und dadurch besonders einfach zum Bedienen. Die berührungsfreie Messung ist insbesondere auch für den Patienten angenehm.

Für die Messung mit einer Vorrichtung basierend auf einem Michelson-Interferometer wird in dessen ersten Strahlengang (Probenarm) das Auge des Patienten gebracht. Die Länge des zweiten Strahlengangs (Referenzarm) dient als Referenzpunkt für die Interferenzmessung. Analog kann die Strahlung auch erst ein Michelson-Interferometer mit variabler Weglängendifferenz durchlaufen und anschliessend auf das Patientenauge gebracht werden.

In Varianten kann statt der obig beschriebenen Interferometer auch ein Tomograph oder ein Sonograph und dergleichen eingesetzt werden. Dem Fachmann ist klar, dass auch andere Interferometertypen statt des Michelson-Interferometers eingesetzt werden können - zum Beispiel ein Mach-Zehnder-Interferometer oder dergleichen.

Für die interferometrische Längenmessung bestehen im Wesentlichen die folgenden beiden Möglichkeiten:
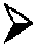 Die Time-Domain (TD) Kohärenzreflektometrie nutzt einen Referenzarm mit variabler Länge und eine Lichtquelle mit kurzer Kohärenzlänge. Zur Abstandsmessung wird die Referenzarmlänge kontinuierlich variiert; sobald die optische Pfadlänge im Referenzarm innerhalb einer Kohärenzlänge einer optischen Pfadlänge im Probenarm entspricht kann Interferenz beobachtet werden. 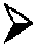
In Fourier-Domain (FD) Kohärenzreflektometrie wird dagegen bei konstanter Referenzarmlänge das Interferenzsignal spektral aufgelöst detektiert; die spektrale Auflösung kann dabei erreicht werden, indem man eine spektral breitbandige Lichtquelle nutzt und das Interferenzsigal beispielsweise mithilfe eines Spektrometers in seine spektralen Komponenten zerlegt (Spectral Domain (SD)-Kohärenzreflektometrie), oder indem man eine schmalbandige Lichtquelle nutzt, welche über ein Frequenzintervall verstimmt werden kann und das Interferenzsignal als Funktion der Quellenfrequenz detektiert. Die Struktur der untersuchten Probe ergibt sich dann aus der Fouriertransformation des spektral aufgelösten Interferenzsignals.

Vorzugsweise ist das Interferometer als Fourierdomäneninterferometer ausgebildet, da diese gegenüber der Time-Domain Interferometrie eine höhere Empfindlichkeit aufweist.

Alternativ kann das Interferometer auch als Time-Domain Interferometrie ausgebildet sein.

Vorzugsweise ist das Interferometer oder zumindest der Strahlengang des Probenarms relativ zum Auge lateral verschiebbar und/oder schwenkbar. Damit können mehrere lateral versetzte und/oder verkippte Abstandsmessungen zu einem Volumenbild verknüpft werden. Dies kann beispielsweise erreicht werden, indem man in den Strahlengang ein Paar galvanometrisch betriebener Scanspiegel einfügt. Für eine Fachperson ist dabei klar ersichtlich, dass eine solche Bewegung der Messachse beispielsweise auch durch eine Bewegung der Fixierhilfe für das Auge oder die mechanische Bewegung der gesamten Anordnung erreicht werden kann.

In Varianten kann auf die laterale Verschiebbarkeit oder Schwenkbarkeit auch verzichtet werden.

Vorzugsweise umfasst die Anordnung eine Schwenkachse, womit der Adapter schwenkbar mit der Vorrichtung verbindbar ist. Damit kann der Adapter bei Bedarf in den Strahlengang der Vorrichtung eingeschwenkt werden und Messungen der zweiten Eigenschaft des Auges durchgeführt werden. Vorteilhaft ist dabei, dass der Adapter stets bereit steht. Die Schwenkachse kann zur Messung der zweiten Eigenschaft und zur Messung der ersten Eigenschaft definierte Schwenkpositionen umfassen. Diese können zum Beispiel durch Einrastungen, vordefiniert sein, womit die Bedienung der Anordnung vereinfacht werden kann. Die aktive Position kann zum Beispiel durch einen Permanentmagneten fixiert werden. Die Schwenkachse kann grundsätzlich beliebig orientiert sein, vorteilhaft ist sie jedoch vertikal angeordnet, da damit Einwirkungen durch die Schwerkraft vermieden werden.

In Varianten kann auf die Schwenkachse auch verzichtet werden.

In einem Verfahren zum Messen von Eigenschaften eines Auges, unter Verwendung einer Anordnung, umfassend einen Adapter und eine Vorrichtung zum Messen einer ersten Eigenschaft des Auges, wobei der Adapter, vorzugsweise lösbar, an der Vorrichtung befestigbar ist, wird zum Messen der zweiten Eigenschaft des Auges der Adapter in einen Strahlengang der Vorrichtung positioniert.

Das Verfahren kann weiter die folgenden Schritte umfassen:
- Messen einer ersten Eigenschaft des Auges zum Erhalten von ersten Messwerten;
- Steuern, insbesondere Positionieren, des Adapters in Abhängigkeit der ersten Messwerte.

Damit können Synergien zwischen der Vorrichtung und dem Adapter erreicht werden. In einem Verfahren zum Ermitteln der Topographie des Auges (Krümmungen etc.) anhand des Reflektionsbilds einer strukturierten Beleuchtung sind Kenntnisse über den Abstand der strukturierten Lichtquelle (Adapter) zur reflektierenden Struktur, also dem Auge, notwendig. Vorzugsweise wird nun zu diesem Zweck die interferometrische Abstandsmessung der Vorrichtung gebraucht, insbesondere da die interferometrische Abstandsmessung besonders präzise ist.

Alternativ kann dieser Abstand auch durch Triangulation oder durch Schnittbildaufnahmen ermittelt etc. werden.

Vorzugsweise wird mit der Vorrichtung eine Länge des Auges bestimmt und anhand der Länge des Auges die Position des Adapters eingestellt und/oder überwacht.

Durch Anbringen des Adapters, zum Beispiel des Reflektometriemoduls, verkürzt sich der Abstand der Anordnung (Vorrichtung und Adapter) zum Auge des Patienten. Um das Risiko einer Kollision zwischen dem Auge des Patienten und dem Adapter zu minimieren, kann eine Steuer- und Analyseeinheit derart gestaltet werden, dass die Abstandsmessung der Vorrichtung kontinuierlich aktiviert wird, und bei Unterschreiten einer kritischen Distanz zum Auge (unter Einbeziehung der Abmessungen des Adapters) ein akustischer, optischer oder mechanischer Warnhinweis ausgegeben wird.

Alternativ kann die Position auch manuell überwacht und nachgestellt werden.

Bevorzugt umfasst die Anordnung eine Positioniervorrichtung zum, vorzugsweise automatischen, Positionieren der Vorrichtung und/oder des Adapters zum Auge.

Die Vorrichtung umfasst vorzugsweise einen motorisierten x-y-z Verstelltisch. Über diesen kann der Messkopf oder aber die ganze Vorrichtung positioniert werden. In diesem Fall kann die Steuereinheit genutzt werden, um anhand der Abstandsinformation der Vorrichtung und/oder der aus der Bildaufnahmeeinheit gewonnenen Information, unter Kenntnis der Abmessungen des Adapters und der Vorrichtung, den Messkopf der Vorrichtungen mit dem Adapter, unter Vermeidung von Kollisionen mit dem Patienten, in eine optimale Messposition zu bringen.

Verfügt die Vorrichtung über eine Positioniervorrichtung, so kann die Steuereinheit auch genutzt werden, um anhand der Abstandsinformation der Vorrichtung und/oder der aus der Bildaufnahmeeinheit gewonnenen Information zwischen sequentiellen Messungen auftretende Abweichungen der Messposition mittels der Positioniervorrichtung auszugleichen und somit eine gewählte Messposition aufrechtzuerhalten.

Zusammenfassend ist festzuhalten, dass die erfindungsgemässe Anordnung aus zwei mechanisch trennbaren Vorrichtungen besteht.

In einer Ausführungsform der Erfindung dient die Vorrichtung der Messung axialer Abstände im Auge und beinhaltet weiter eine Abbildungsoptik, eine Bildaufnahmeeinheit, und eine Steuer- und Analyseeinheit. Vorzugsweise umfasst die Vorrichtung eine Schnittstelle, zum Beispiel eine USB-Schnittstelle oder eine drahtlose Verbindung oder dergleichen, mit einem PC (Personal Computer). Damit kann die Steuerung der Messung sowie die Auswertung der Daten über den PC erfolgen. Die Vorrichtung umfasst aber bevorzugt ebenfalls eine Elektronik, welche schnelle Regelkreise und eine Datenerfassung enthält. In Varianten können sämtliche Funktionalitäten auch in einem All-in-one-Gerät realisiert sein, wobei die Vorrichtung die Steuerung und die Auswertung der Daten übernimmt. In diesem Fall könnte auf eine Verbindung der Vorrichtung mit einem separaten PC verzichtet werden.

Der Adapter umfasst mindestens eine Einheit zur strukturierten Beleuchtung des Auges, welche zwischen der Vorrichtung und dem Auge angebracht werden kann. Die mindestens eine Lichtquelle welche der mindestens einen Einheit zur strukturierten Beleuchtung des Auges dient, kann sich in der Vorrichtung und/oder im Adapter befinden.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine schematische Darstellung einer Vorrichtung zur interferometrischen Messung von Längen in Augen, nach dem Stand der Technik;
- Fig. 2: eine schematische Darstellung eines Querschnitts einer Vorrichtung zum interferometrischen Längenmessen, entlang einer optischen Achse;
- Fig. 3: eine schematische Frontansicht einer Vorrichtung gemäss Figur 2 in Richtung der optischen Achse;
- Fig. 4: eine schematische Darstellung eines Querschnitts eines Adapters zur Placidoringprojektion, entlang der optischen Achse;
- Fig. 5: eine schematische Schrägansicht des Adapters gemäss Figur 4;
- Fig. 6: eine schematische Rückansicht eines Adapters gemäss Figur 4 in Richtung der optischen Achse;
- Fig. 7: eine schematische Darstellung eines Querschnitts durch eine Anordnung umfassend eine Vorrichtung zum interferometrischen Längenmessen und eines Adapters, entlang einer optischen Achse;
- Fig. 8: eine schematische Darstellung eines Querschnitts eines Adapters, im Wesentlichen gemäss Figur 4, umfassend zusätzliche Lichtleiter;
- Fig. 9: eine schematische Schrägansicht des Adapters gemäss Figur 8.

Grundsätzlich sind in den Figuren gleiche Teile mit gleichen Bezugszeichen versehen.

### Wege zur Ausführung der Erfindung

Die Vorrichtung zur Messung axialer Abstände im Auge kann beispielsweise auf dem Time-Domain Kohärenzreflektometrieverfahren beruhen, wie dies auch im Biometer Lenstar LS 900 der Firma Haag-Streit AG umgesetzt ist und in EP 1946039 B1 ausführlich beschrieben wird.

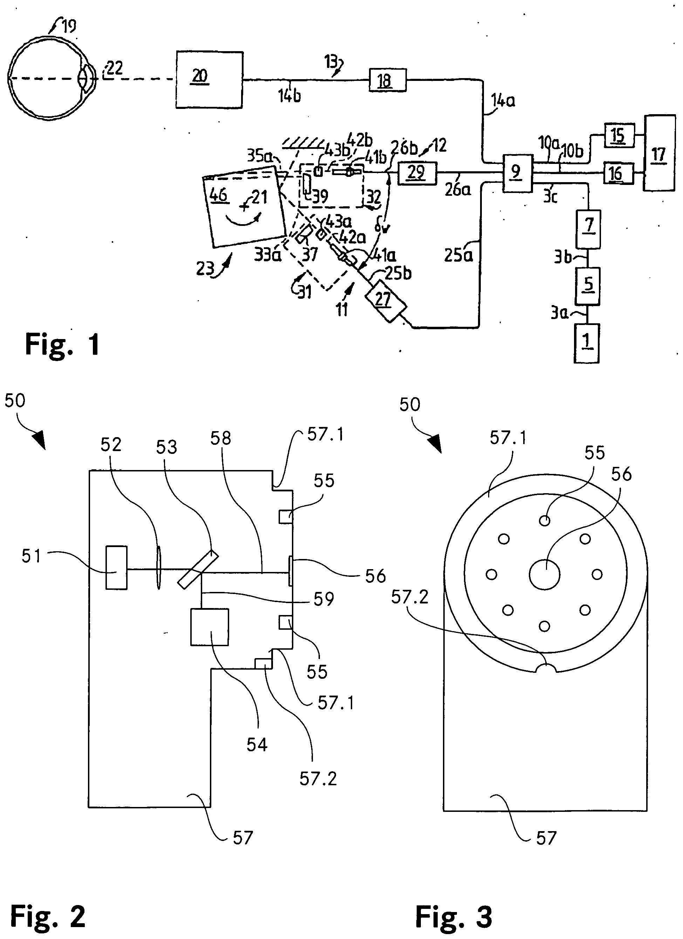

Fig. 1 zeigt schematisch einen möglichen Aufbau dieser Vorrichtung. Die Erfindung ist jedoch keineswegs auf dieses Verfahren beschränkt, sondern lässt sich beispielsweise ebenso gut mit Spektraldomänen- oder Swept-Source-Kohärenzreflektometrie umsetzen.

Die Vorrichtung kann dabei auch über mehrere Messbereiche und/oder mehrere Interferometer verfügen.

In Figur 1 ist ein Ausführungsbeispiel der erfindungsgemässen Vorrichtung mit einer "Michelson-Interferometer"-artigen optischen Anordnung dargestellt. Die Vorrichtung hat in dieser Anordnung eine Strahlungsquelle 1, deren Strahlung eine kurze Kohärenzlänge hat. Die Kohärenzlänge ist wesentlich kürzer als die auszumessenden Dicken und Weglängen, und liegt im Bereich von 2 µm bis 100 µm. Wobei normalerweise in einem Bereich von 10 µm bis 20 µm gearbeitet wird. Je kürzer die Kohärenzlänge desto genauer kann gemessen werden. Man wird deshalb eine Strahlungsquelle 1 mit einer möglichst breitbandigen Ausgangsstrahlung wählen. Als Strahlungsquelle 1 kann eine Superlumineszenzdiode (SLD), eine Licht emittierende Diode (Light Emitting Diode LED), eine Weisslichtquelle bzw. eine Lichtquelle mit verstärkter spontaner Emission (Amplified Spontaneous Emission Lichtquelle (ASE), z. B. die Strahlung eines Dioden gepumpten Festkörpers) verwendet werden. Diese Strahlung wird in eine Monomode-Faser 3a eingekoppelt.

Die Monomodefaser 3a wird zu einem Strahlungsabschwächer 5 geführt. Der Strahlungsabschwächer 5 kann z. B. eine Verlustspleissung zwischen zwei nicht dargestellten Monomodefasern sein. Mit dem Strahlungsabschwächer 5 kann eine Abschwächung der von den unten beschriebenen Referenzarmen und vom Messarm in Richtung zur Strahlungsquelle 1 zurückreflektierten Strahlung vorgenommen werden. In die Strahlungsquelle 1 zurückreflektierte Strahlung könnte beispielsweise bei einer SLD, einer ASE oder bei einem Dioden gepumpten Festkörper das Emissionsverhalten negativ beeinflussen. Der Strahlungsabschwächer 5 dient somit zur Minimierung dieses negativen Einflusses. Vom Strahlungsabschwächer 5 wird die nun eventuell abgeschwächte Strahlung der Strahlungsquelle 1 in eine Monomodefaser 3b zu einem Polarisationskontroller 7 geführt. Mit Hilfe des Polarisationskontrollers 7 wird der Polarisationszustand der Strahlung eingestellt, welche den Kontroller durchläuft. Der Strahlungsabschwächer 5 und der Polarisationskontroller 7 sind optional und sind für die erfindungsgemässe Vorrichtung nicht zwingend notwendig.

Die Strahlung der Strahlungsquelle 1 wird weiter mittels einer Monomodefaser 3c auf einen 3 x 3 - Monomodefaserkoppler 9 geführt. Mit Hilfe des Monomodefaserkopplers 9 wird die Strahlung der Strahlungsquelle 1 in zwei Referenzarme 11 und 12 und in einen Messarm 13 aufgeteilt. Die beiden restlichen Ausgänge des 3 x 3 - Monomodefaserkopplers 9 sind mittels jeweils einem Strahlungsleiter 10a und 10b mit je einem Detektor 15 bzw. 16 verbunden, welcher seinerseits signaltechnisch mit einer Detektionselektronik 17 verbunden ist. Die Detektoren 15 und 16 messen die Interferenzen der von Messobjekt 19 reflektierten Strahlung mit reflektierter Strahlung in Referenzarm 11 und/oder 12.

Der Messarm 13 hat eine Monomodefaser 14a, deren eines Ende mit dem Monomodefaserkoppler 9 verbunden ist. Das andere Ende der Monomodefaser 14a ist mit einem Polarisationskontroller 18 verbunden, von dem eine weitere Monomodefaser 14b zu einem unten im Detail beschriebenen Messkopf 20 führt. Vom Messkopf 20 geht dann die Strahlung als Freiraumstrahl 22 zum Messobjekt 19 (hier einem Auge).

Die Referenzarme 11 und 12 haben entsprechend der zwei Messbereiche eine unterschiedliche optische Grundweglänge. In beiden Referenzarmen 11 und 12 wirkt ein und dasselbe, um eine Elementachse 21 rotierbares Weglängenvariationselement 23.

Die Referenzarme 11 und 12 sind über je eine Monomodefaser 25a bzw. 26a mit dem 3 x 3 - Monomodefaserkoppler 9 verbunden. Das jeweils dem Monomodefaserkoppler 9 abgewandte Ende der Monomodefaser 25a bzw. 26a ist mit einem Polarisationskontroller 27 bzw. 29 verbunden. Vom jeweiligen Polarisationkontroller 27 bzw. 29 führt je eine Monomodefaser 25b bzw. 26b zu einer optischen Einheit 31 bzw. 32, welche die in der Monomodefaser 25b bzw. 26b u.a. geführte Strahlung jeweils in einen Freiraumstrahl 33a bzw. 35a verwandelt. Die beiden Freiraumstrahlen 33a und 35a werden unter einem gegenseitigen Versetzungswinkel W auf das Weglängenvariationselement 23 geführt und werden vom Weglängenvariationselement 23, sofern dieses, wie unten beschrieben, eine bestimmte Drehposition hat, auf jeweils einen Spiegel 37 bzw. 39 in der optischen Einheit 31 bzw. 32 geführt und von diesem wieder über das Weglängenvariationselement 23 zurück in die Monomodefaser 25b bzw. 26b reflektiert. Der Versetzungswinkel W ist bis eine Winkeltoleranz halb so gross wie ein Eckenwinkel δ des Weglängenvariationselements 23. Die gewählte Winkeltoleranz beeinflusst die zur Verfügung stehende Weglängenvariationslänge. Je grösser die Winkeltoleranz gewählt wird, desto kleiner wird die Weglängenvariationslänge. Da in dem hier ausgesuchten Beispiel ein quadratischer Querschnitt des Weglängenvariationselements 23 gewählt worden ist, ist der Versetzungswinkel w von 45 °. Das hier gewählte Weglängenvariationselement 23 hat einen Brechungsindex von 1,5 und eine Kantenbreite lk von beispielsweise 40 mm; man wird deshalb mit einer Winkeltoleranz von ± 5 ° arbeiten.

Jede optische Einheit 31 bzw. 32 hat eine Ferrule 41a bzw. 41b zum Fassen des dem Polarisationskontroller 27 bzw. 29 abgewandten Endes der Monomodefaser 25b bzw. 26b. Nach der Ferrule 41a bzw. 41b breitet sich die in der Monomodefaser 25b bzw. 26b geführte Strahlung als Freiraumstrahl 42a bzw. 42b aus und wird mittels einer Linseneinheit 43a bzw. 43b zum Freiraumstrahl 33a bzw. 35a kollimiert. Die Linseneinheit 43a bzw. 43b kann nun ein Achromat, eine Einzellinse oder auch ein Linsensystem sein. Der vom jeweiligen Spiegel 37 bzw. 39 rückreflektierte Strahl wird von der Linseneinheit 43a bzw. 43b über die Ferrule 41a bzw. 41b in die Monomodefaser 25b bzw. 26b eingekoppelt.

Die Figur 2 zeigt eine Vorrichtung 50, vorliegend geeignet zur axialen Längenmessung mit einem Gehäuse 57 und einem metallischen Absatz 57.1 zur Aufnahme eines Adapters 60 mit Magneten 66 (siehe unten), entlang einer optischen Achse 58. Im Absatz 57.1 ist weiter eine im axialen Querschnitt halbkreisförmige Aussparung 57.2 zur eindeutigen Positionierung des Adapters 60 ausgebildet.

Die Vorrichtung 50 umfasst weiter eine Abbildungsoptik 52 und Bildaufnahmeeinheit 51, welche über einen Strahlteiler 53 mit dem Strahlengang 58 des Interferometers 54 überlagert werden. Als Lichtquelle dienen hier konzentrisch um den Strahlengang 58 angeordnete LED's 55. In einem Biometer dient diese Anordnung zur fotographischen Frontalabbildung des Auges. Dies kann beispielsweise zur Dokumentation von biometrischen Untersuchungen genutzt werden.

Im Rahmen dieser Ausführungsform der Erfindung können diese Komponenten auch genutzt werden, um die Reflektion der strukturierten Beleuchtung durch das Auge zu detektieren. Damit kann die Aufnahmeeinheit bestehend aus der Abbildungsoptik 52 und der Bildaufnahmeeinheit 51 sowohl dem Detektieren der Augenlängen (vorliegend die erste Eigenschaft) assistieren, als auch zum Detektieren der Topographie (vorliegend die zweite Eigenschaft) eingesetzt werden, womit die Anordnung besonders kostengünstig wird.

Vorteilhaft ist eine Anordnung in welcher die Achse zur Abstandsmessung variabel ist, so dass mehrere lateral versetzte und/oder verkippte Abstandsmessungen zu einem Volumenbild verknüpft werden können. Dies kann beispielsweise erreicht werden, indem man in den Strahlengang 59 ein Paar galvanometrisch betriebene Scanspiegel einfügt. Für eine Fachperson ist es dabei klar ersichtlich, dass eine solche Bewegung der Messachse beispielsweise auch durch eine Bewegung der Fixierhilfe für das Auge oder die mechanische Bewegung der gesamten Anordnung erreicht werden kann.

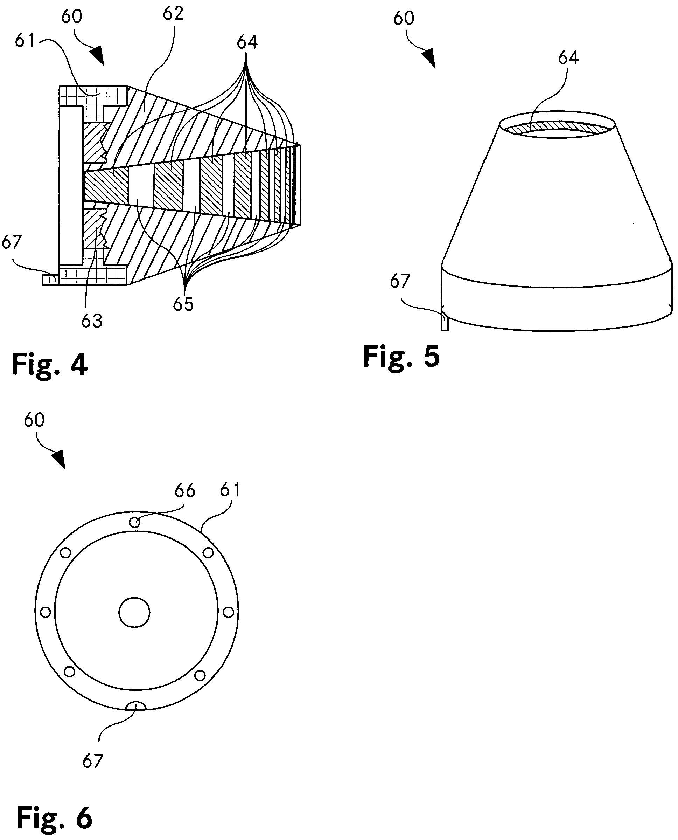

Figur 3 zeigt eine schematische Frontansicht einer Vorrichtung 50 gemäss Figur 2 in Richtung der optischen Achse 58. Diese zeigt, dass der Absatz 57.1 als kreisringförmiger Absatz ausgebildet ist und dieser eine halbkreisförmiger Aussparung 57.2 umfasst, womit der Adapter 60, mit ringförmigem Flansch 61 und einer entsprechend geformten Ausbuchtung 67 am Flansch 61 (siehe Figur 6), in genau einer Orientierung an der Vorrichtung 50 befestigt werden kann. Dem Fachmann sind auch weitere Möglichkeiten bekannt, wie die eindeutige Orientierung des Adapters 60 an der Vorrichtung 50 erreicht werden kann.

Die Vorrichtung 50 umfasst LED's 55, welche ringförmig angeordnet sind. In der vorliegenden Figur 3 sind 8 LED's dargestellt, dem Fachmann ist aber klar, dass auch mehr oder weniger LED's vorgesehen sein können. Insbesondere sind bevorzugt zwischen 8 und 16 LED's, besonders bevorzugt 12 LED's kreisförmig angeordnet. Es hat sich als zweckmässig erwiesen, die Lichtquellen zur vereinfachten Datenanalyse in einem gleichförmig auf einer oder mehreren Kreislinien, die konzentrisch mit der Symmetrieachse der axialen Messung angeordnet sind, zu verteilen.

Der durch die LED's 55 gebildete Kreis weist einen kleineren Durchmesser auf als der Absatz 57.1 und ist konzentrische zum Absatz 57.1 orientiert. In der Mitte des LED-Kreises befindet sich die Öffnung für den Detektor, respektive die Bildaufnahmeeinheit 51 und das Interferometer 54. Statt der LED's können auch Laserdioden, die Licht im sichtbaren oder infraroten Spektralbereich emittieren, eingesetzt werden.

Figur 4 zeigt eine schematische Darstellung eines Querschnitts eines Adapters 60 zur Placidoringprojektion, in Richtung der optischen Achse. Der Adapter 60 ist als mechanischer Aufsatz für die Vorrichtung 50 ausgebildet und nutzt die LED's 55 der Vorrichtung 50 als Lichtquelle, das heisst, der Adapter 60 umfasst in dieser Ausführungsform keine eigene Lichtquelle.

Der Adapter 60 weist im Wesentlichen die Form eines Kreiskegelstumpfes auf, welche an der Grundfläche einen kurzen, geraden Zylinderfortsatz umfasst. Bei einer Messung ist das schmale Ende des Adapters 60, respektive des Kreiskegelstumpfs dem zu vermessenden Auge zugewandt. Der Adapter 60 umfasst am Zylinderfortsatz einen Flansch 61 oder Kragen, welcher als Gegenstück zum Absatz 57.1 der Vorrichtung 50 ausgebildet ist. Zusätzlich umfasst der Flansch 61 eine im radialen Querschnitt halbkreisförmige Ausbuchtung 68, welche bei der Montage in die halbkreisförmige Aussparung 57.2 der Vorrichtung zu liegen kommt. Innerhalb des Zylinderfortsatzes ist ein kreisringförmiges fresnellinsenartigen Profil 63 angeordnet, welches sich unmittelbar an einen hohlkegelförmigen Lichtleiter 62 anschliesst. Der Lichtleiter 62 ist vorzugsweise aus PMMA (Polymethylmethacrylat) geformt, da PMMA besonders einfach bearbeitet werden kann und kostengünstig ist. Selbstverständlich können auch weitere Stoffe eingesetzt werden, welche bei gegebener Lichtfrequenz lichtleitend sind, zum Beispiel Glas oder andere Kunststoffe. Der Lichtleiter 62 ist vorzugsweise in eine metallische Umhüllung gefasst. Statt der metallischen Umhüllung kann der hohlkegelförmige Lichtleiter auch lediglich aussen beschichtet sein, zum Beispiel mit einer lichtreflektierenden Beschichtung wie zum Beispiel einer Metallbeschichtung oder einem Farblack.

Das Profil 63 dient einer effizienteren Einkopplung des Lichts von den LED's 55 der Vorrichtung in den Lichtleiter 62. Das Profil 63 ist jedoch für die Funktion des Reflektometriemoduls nicht zwingend erforderlich, so kann darauf auch verzichtet werden, insbesondere wenn eine hinreichend starke und homogene Beleuchtungseinrichtung vorliegt.

Der Lichtleiter 62 ist als Hohlkegel ausgebildet und weist wiederum einen zum Lichtleiter 62 koaxialen kegelstumpfförmigen Hohlraum oder eine konische Aussparung auf. Das heisst, der Hohlraum oder die Aussparung und der Aussenmantel des Lichtleiters 62 weisen eine gemeinsame Symmetrieachse auf. Die Grundfläche des kegelstumpfförmigen Hohlraums liegt dabei aber der Grundfläche des Lichtleiters 62 gegenüber und bildet ein distales Ende des Adapters 60, welches bei einer Messung auf das Auge gerichtet ist. Der Hohlraum weist nun alternierend opake Bereiche 64 und lichtdurchlässige Bereich 65 auf. Das Licht wird nun über diese Bereiche 64, 65 ringförmig in axialer Richtung auf ein zu vermessendes Auge (nicht dargestellt) gestrahlt.

Figur 5 zeigt eine schematische Schrägansicht des Adapters 60 gemäss Figur 4. Ersichtlich ist dabei die im Wesentlichen kreiskegelstumpfartige Form des Adapters 60.

Figur 6 zeigt eine schematische Rückansicht eines Adapters gemäss Figur 4 in Richtung der optischen Achse, das heisst, die Unteransicht des Kreiszylinderfortsatzes. Ersichtlich ist dabei der Flansch 61, welcher im Randbereich eine halbkreisförmige Ausbuchtung 67 aufweist. Der Flansch 61 umfasst mehrere, vorliegend sieben, Permanentmagnete 66, über welche der Adapter 60 am metallischen Absatz 57.1 der Vorrichtung 50 gehalten werden kann. Mittig ist schliesslich die Öffnung ersichtlich, welche in montiertem Zustand mit der Öffnung 56 der Vorrichtung kommuniziert und so eine Aufnahme und Analyse der Bilddaten ermöglicht.

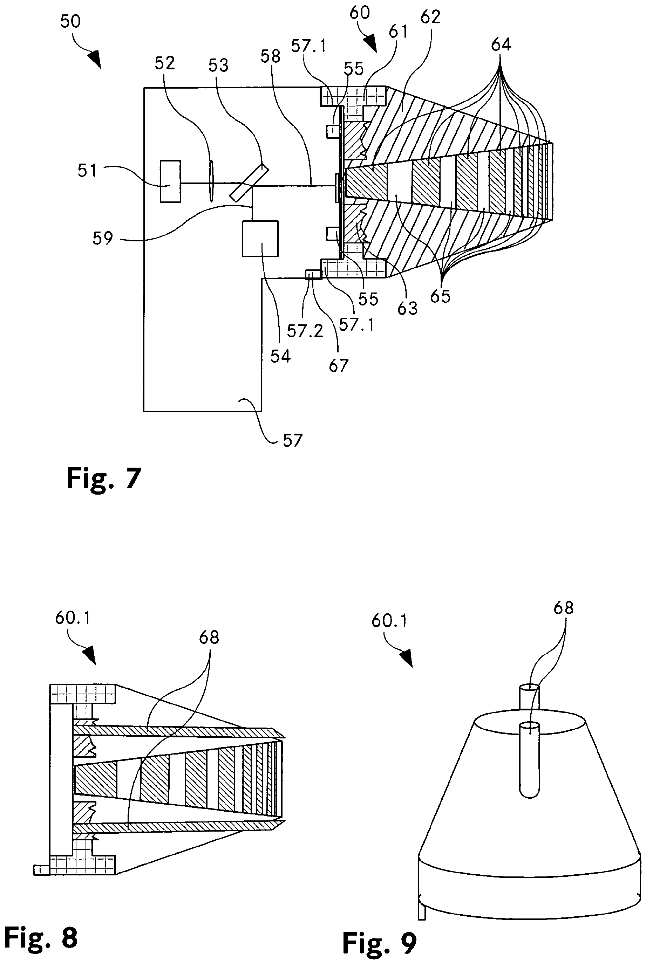

Figur 7 zeigt eine schematische Darstellung eines Querschnitts durch eine Anordnung umfassend eine Vorrichtung 50 zum interferometrischen Längenmessen und einen Adapter 60, entlang einer optischen Achse. Die Vorrichtung 50 entspricht dabei der Figur 2 und der Adapter 60 der Figur 4. Der Flansch 61 des Adapters 60 ist dabei auf den Absatz 57.1 der Vorrichtung 50 geschoben, so dass die Ausbuchtung 67 in der Aussparung 57.2 liegt und den Adapter 60 bezüglich einer Rotation um seine Symmetrieachse fixiert. Derweilen ist der Adapter 60 über die Permanentmagnete 66 am metallischen Absatz 57.1 lösbar gehalten.

Figur 8 zeigt eine schematische Darstellung eines Querschnitts eines Adapters 60, im Wesentlichen gemäss Figur 4, aber mit zwei zusätzliche Lichtleiter 68. Diese sind stabförmig ausgebildet und im Wesentlichen parallel zur Symmetrieachse, jedoch geringfügig in distaler Richtung hin zur Symmetrieachse geneigt innerhalb Adapters 60 angeordnet. Mit den Lichtleiter 68 kann Licht von den LED's 55 an die dem Auge zugewandten Seite des Adapters leiten. Der augenseitiger radialer Abstand zur Messachse bei normalem Messabstand zum Auge ist vorzugsweise so gross gewählt, dass die direkte Reflektion über die Hornhautvorderseite die Bildaufnahmeeinheit nicht erreicht, dagegen aber das Auge durch diffuses Streulicht indirekt beleuchtet wird. Figur 9 zeigt schliesslich eine schematische Schrägansicht des Adapters gemäss Figur 8.

Die Vorrichtung verfügt wahlweise über einen Signalgeber (nicht dargestellt), welcher der Steuer- und Analyseeinheit signalisiert, ob der Adapter mit der Vorrichtung verbunden ist. Dieser Signalgeber kann beispielsweise auf einem mechanischen Schalter, einer Lichtschranke oder einem magnetischen Schalter basieren. Die Steuereinheit kann das Signal nutzen, um die jeweils verfügbaren Messfunktionen zu erkennen und auszuführen, sowie die Lichtquellen und Bildaufnahmeeinheit kontextspezifisch anzusteuern. Die in Figur 2 gezeigte Ausführung kann beispielsweise bei aufgesetztem Adapter (Reflektometriemodul) eine Hornhauttopographiemessung und bei entferntem Adapter eine Keratometriemessung vornehmen, ohne vom Bediener eine entsprechende Vorauswahl der Messfunktion zu erwarten. Dabei können Beleuchtungsintensität und Detektionssensitivität automatisch auf den für die jeweilige Messfunktion optimalen Wert eingestellt werden.

Zusammenfassend ist festzustellen, dass erfindungsgemäss eine Anordnung geschaffen wird, welche bei geringen Herstellungskosten in einfacher Weise entweder zur Vermessung einer Länge oder einer Oberfläche eines Auges eingesetzt werden kann.

## Patentansprüche

1. Anordnung (50, 60) zum Messen von Eigenschaften eines Auges, umfassend einen Adapter (60) und eine Vorrichtung (50) zum Messen einer ersten geometrischen Eigenschaft eines Auges, wobei der Adapter (60), vorzugsweise lösbar, an der Vorrichtung (50) befestigbar ist, wobei mit dem an der Vorrichtung (50) befestigten Adapter (60), eine zweite zusätzliche geometrische Eigenschaft des Auges messbar ist, wobei die erste Eigenschaft einen Abstand in einem Auge umfasst, welche bei abgenommenem Adapter gemessen werden kann und die zweite Eigenschaft zumindest eine Topographie oder eine Krümmung des Auges umfasst.

2. Anordnung (50, 60) nach Anspruch 1, **dadurch gekennzeichnet, dass** die erste Eigenschaft mindestens eine der nachfolgenden Längen umfasst: Hornhautdicke, Vorderkammertiefe, Linsendicke, Augenlänge.

3. Anordnung (50, 60) nach Anspruch 1 oder 2, **dadurch gekennzeichnet, dass** der Adapter (60) Kopplungsmittel (61, 66, 67) umfasst, womit der Adapter (60) an der Vorrichtung (50) befestigbar ist, wobei die Kopplungsmittel (61, 66, 67) vorzugsweise einen Magneten (66) umfassen.

4. Anordnung (50, 60) nach einem der Ansprüche 1 bis 3, **dadurch gekennzeichnet, dass** der Adapter (60) einen ersten Lichtleiter (62) zur strukturierten Beleuchtung des Auges umfasst.

5. Anordnung (50, 60) nach einem der Ansprüche 1 bis 4, **dadurch gekennzeichnet, dass** der Adapter (60) einen zweiten Lichtleiter (68) zur diffusen oder indirekten Beleuchtung des Auges umfasst.

6. Anordnung (50, 60) nach einem der Ansprüche 1 bis 5, **dadurch gekennzeichnet, dass** die Vorrichtung (50) einen Detektor, vorzugsweise eine Bildaufnahmeeinheit, umfasst, womit bei, an der Vorrichtung (50) befestigtem Adapter (60), eine zweite Eigenschaft des Auges messbar ist.

7. Anordnung (50, 60) nach einem der Ansprüche 1 bis 6, **dadurch gekennzeichnet, dass** die Vorrichtung (50) eine Lichtquelle umfasst und der Adapter (60) einen ersten Lichtleiter umfasst, so dass bei an der Vorrichtung (50) befestigtem Adapter (60) Licht von der Lichtquelle über den ersten Lichtleiter zum Auge leitbar ist.

8. Anordnung (50, 60) nach einem der Ansprüche 1 bis 7, **dadurch gekennzeichnet, dass** der Adapter (60) einen zweiten Lichtleiter umfasst, womit Licht von einer Lichtquelle der Vorrichtung (50) oder des Adapters (60) zur diffusen und/oder indirekten Beleuchtung auf das Auge führbar ist.

9. Anordnung (50, 60) nach einem der Ansprüche 1 bis 8, **dadurch gekennzeichnet, dass** mit dem Adapter (60) eine strukturierte Beleuchtung, insbesondere in der Form von Punkten und/oder Ringen, auf dem Auge erreichbar ist.

10. Anordnung (50, 60) nach einem der Ansprüche 1 bis 9, **dadurch gekennzeichnet, dass** die Vorrichtung (50) einen Sensor zum Erkennen des Adapters (60) umfasst.

11. Anordnung (50, 60) nach Anspruch 10, **dadurch gekennzeichnet, dass** die Vorrichtung (50) eine Steuereinheit umfasst, womit in Abhängigkeit eines Messwertes des Sensors ein Messmodus für die erste Eigenschaft des Auges respektive ein Messmodus für die zweite Eigenschaft des Auges schaltbar ist.

12. Anordnung (50, 60) nach einem der Ansprüche 1 bis 11, **dadurch gekennzeichnet, dass** die Vorrichtung (50) ein Interferometer, vorzugsweise ein Michelson-Interferometer umfasst.

13. Anordnung (50, 60) nach Anspruch 12, **dadurch gekennzeichnet, dass** das Interferometer als Fourierdomäneninterferometer ausgebildet ist.

14. Anordnung (50, 60) nach Anspruch 12 oder 13, **dadurch gekennzeichnet, dass** das Interferometer relativ zum Auge lateral verschiebbar und/oder schwenkbar ist.

15. Anordnung (50, 60) nach einem der Ansprüche 1 bis 14, **dadurch gekennzeichnet, dass** sie eine Positioniervorrichtung zum, vorzugsweise automatischen, Positionieren der Vorrichtung (50) und/oder des Adapters (60) zum Auge umfasst.

16. Anordnung (50, 60) nach einem der Ansprüche 1 bis 15, **dadurch gekennzeichnet, dass** sie eine Schwenkachse umfasst, womit der Adapter (60) schwenkbar mit der Vorrichtung (50) verbindbar ist.

17. Verfahren zum Messen von Eigenschaften eines Auges, unter Verwendung einer Anordnung (50, 60) nach einem der Ansprüche 1 bis 16, wobei zum Messen der zumindest einen Topographie oder einen Krümmung des Auges der Adapter (60) in einen Strahlengang der Vorrichtung (50) positioniert wird.

18. Verfahren nach Anspruch 17, **gekennzeichnet durch** die folgenden Schritte:
a. Messen einer ersten Eigenschaft des Auges zum Erhalten von ersten Messwerten;
b. Steuern, insbesondere Positionieren, des Adapters (60) in Abhängigkeit der ersten Messwerte.

19. Verfahren nach Anspruch 18, **dadurch gekennzeichnet, dass** mit der Vorrichtung (50) ein Abstand der Vorrichtung (50) zum Auge bestimmt wird und anhand des Abstands die Position des Adapters (60) eingestellt und/oder überwacht wird.

## Claims

1. Arrangement (50, 60) for measuring properties of an eye, comprising an adapter (60) and an apparatus (50) for measuring a first geometric property of an eye, wherein the adapter (60) is attachable, preferably releasably, to the apparatus (50), **characterized in that**, when the adapter is attached to the apparatus, a second, additional geometric property of the eye is measurable, wherein the first property comprises a distance in an eye and the second property comprises at least a topography or a curvature of the eye.

2. Arrangement (50, 60) according to Claim 1, **characterized in that** the first property comprises at least one of the following lengths: cornea thickness, anterior chamber depth, lens thickness, eye length.

3. Arrangement (50, 60) according to one of Claims 1 or 2, **characterized in that** the adapter (60) comprises coupling means (61, 66, 67), with which the adapter (60) is attachable to the apparatus (50), wherein the coupling means (61, 66, 67) preferably comprise a magnet (66).

4. Arrangement (50, 60) according to one of Claims 1 to 3, **characterized in that** the adapter (60) comprises a first optical waveguide (62) for structured illumination of the eye.

5. Arrangement (50, 60) according to one of Claims 1 to 4, **characterized in that** the adapter (60) comprises a second optical waveguide (68) for diffuse or indirect illumination of the eye.

6. Arrangement (50, 60) according to one of Claims 1 to 5, **characterized in that** the apparatus (50) comprises a detector, preferably an image recording unit, with which, when the adapter (60) is attached to the apparatus (50), a second property of the eye is measurable.

7. Arrangement (50, 60) according to one of Claims 1 to 6, **characterized in that** the apparatus (50) comprises a light source and the adapter (60) comprises a first optical waveguide, such that, when the adapter (60) is attached to the apparatus (50), light from the light source is guidable to the eye via the first optical waveguide.

8. Arrangement (50, 60) according to one of Claims 1 to 7, **characterized in that** the adapter (60) comprises a second optical waveguide, with which light from a light source of the apparatus (50) or of the adapter (60) is guidable to the eye for diffuse and/or indirect illumination.

9. Arrangement (50, 60) according to one of Claims 1 to 8, **characterized in that** the adapter (60) can be used to achieve structured illumination, in particular in the form of points and/or rings, on the eye.

10. Arrangement (50, 60) according to one of Claims 1 to 9, **characterized in that** the apparatus (50) comprises a sensor for detecting the adapter (60).

11. Arrangement (50, 60) according to Claim 10, **characterized in that** the apparatus (50) comprises a control unit, with which, in dependence on a measurement value of the sensor, a measurement mode for the first property of the eye and a measurement mode for the second property of the eye are switchable.

12. Arrangement (50, 60) according to one of Claims 1 to 11, **characterized in that** the apparatus (50) comprises an interferometer, preferably a Michelson interferometer.

13. Arrangement (50, 60) according to Claim 12, **characterized in that** the interferometer is configured as a Fourier domain interferometer.

14. Arrangement (50, 60) according to Claim 12 or 13, **characterized in that** the interferometer is pivotable and/or movable laterally with respect to the eye.

15. Arrangement (50, 60) according to one of Claims 1 to 14, **characterized in that** it comprises a positioning apparatus for positioning, preferably automatically, the apparatus (50) and/or the adapter (60) with respect to the eye.

16. Arrangement (50, 60) according to one of Claims 1 to 15, **characterized in that** it comprises a pivot pin, with which the adapter (60) is pivotably connectable to the apparatus (50)..

17. Method for measuring properties of an eye using an arrangement (50, 60) according to one of Claims 1 to 16, comprising an adapter (60) and an apparatus (50) for measuring a distance in an eye, wherein the adapter (60) is attachable, preferably releasably, to the apparatus (50), and wherein, when the adapter is attached to the apparatus, at least a topography or a curvature of the eye is measurable and wherein the adapter (60) is positioned in a beam path of the apparatus (50) for measuring at least a topography or a curvature of the eye.

18. Method according to Claim 17, **characterized by** the following steps:
a. measuring a first property of the eye for obtaining first measurement values;
b. controlling, in particular positioning, the adapter (60) in dependence on the first measurement values.

19. Method according to Claim 18, **characterized in that** the apparatus (50) is used to determine a distance of the apparatus (50) from the eye and the position of the adapter (60) is set and/or monitored on the basis of said distance.

## Revendications

1. Appareillage (50, 60) destiné à la mesure des propriétés d'un oeil, comprenant un adaptateur (60) et un dispositif (50) en vue de la mesure d'une première propriété géométrique d'un oeil, l'adaptateur (60) pouvant être fixé, de préférence de manière amovible, au dispositif (50), une deuxième propriété géométrique supplémentaire de l'oeil pouvant être mesurée au moyen de l'adaptateur (60) fixé au dispositif (50), la première propriété comprenant un espacement dans un oeil, laquelle peut être mesurée quand l'adaptateur est retiré, et la deuxième propriété comprenant au moins une topographie ou une courbure de l'oeil.

2. Appareillage {50, 60) selon la revendication 1, **caractérisé en ce que** la première propriété comprend au moins l'une des longueurs suivantes : l'épaisseur de la cornée, la profondeur de la chambre antérieure, l'épaisseur du cristallin, la longueur de l'oeil.

3. Appareillage (50, 60) selon la revendication 1 ou 2, **caractérisé en ce que** l'adaptateur (60) comprend des moyens de couplage (61, 66, 67) par l'intermédiaire desquels l'adaptateur (60) peut être fixé au dispositif (50), les moyens de couplage (61, 66, 67) comprenant de préférence un aimant (66).

4. Appareillage (50, 60) selon l'une des revendications 1 à 3, **caractérisé en ce que** l'adaptateur (60) comprend un premier conduit de lumière (62) qui est destiné à l'éclairage structuré de l'oeil.

5. Appareillage (50, 60) selon l'une des revendications 1 à 4, **caractérisé en ce que** l'adaptateur (60) comprend un deuxième conduit de lumière (68) qui est destiné à l'éclairage diffus ou indirect de l'oeil.

6. Appareillage (50, 60) selon l'une des revendications 1 à 5, **caractérisé en ce que** le dispositif (50) comprend un détecteur, de préférence une unité d'imagerie, au moyen de laquelle une deuxième propriété de l'oeil peut être mesuré, quand l'adaptateur (60) est fixé au dispositif (50).

7. Appareillage (50, 60) selon l'une des revendications 1 à 6, **caractérisé en ce que** le dispositif (50) comprend une source de lumière et l'adaptateur (60) comprend un premier conduit de lumière, de telle sorte que de la lumière peut être dirigée depuis la source de lumière jusqu'à l'oeil par l'intermédiaire du premier conduit de lumière, quand l'adaptateur (60) est fixé au dispositif (50).

8. Appareillage (50, 60) selon l'une des revendications 1 à 7, **caractérisé en ce que** l'adaptateur (60) comprend un deuxième conduit de lumière, au moyen duquel de la lumière peut être dirigée depuis une source de lumière du dispositif (50) ou de l'adaptateur (60) en vue de l'éclairage diffus et/ou indirect sur l'oeil.

9. Appareillage (50, 60) selon l'une des revendications 1 à 8, **caractérisé en ce qu'**un éclairage structuré, lequel se présente en particulier sous la forme de points ou et/ou d'anneaux, peut être atteint sur l'oeil au moyen de l'adaptateur (60).

10. Appareillage (50, 60) selon l'une des revendications 1 à 9, **caractérisé en ce que** le dispositif (50) comprend un capteur destiné à la détection de l'adaptateur (60).

11. Appareillage (50, 60) selon la revendication 10, **caractérisé en ce que** le dispositif (50) comprend une unité de commande, au moyen de laquelle un mode de mesure peut être commuté pour la première propriété de l'oeil, respectivement un mode de mesure peut être commuté pour la deuxième propriété de l'oeil, en fonction d'une valeur de mesure du capteur.

12. Appareillage (50, 60) selon l'une des revendications 1 à 11, **caractérisé en ce que** le dispositif (50) comprend un interféromètre, de préférence un interféromètre de Michelson.

13. Appareillage (50, 60) selon la revendication 12, **caractérisé en ce que** l'interféromètre est conçu sous la forme d'un spectromètre par transformée de Fourier.

14. Appareillage (50, 60) selon la revendication 12 ou 13, **caractérisé en ce que** l'interféromètre peut être décalé et/ou pivoté de manière latérale par rapport à l'oeil.

15. Appareillage (50, 60) selon l'une des revendications 1 à 14, **caractérisé en ce que** ledit appareillage comprend un dispositif de positionnement destiné au positionnement, de préférence automatique, du dispositif (50) et/ou de l'adaptateur (60) vis-à-vis de l'oeil.

16. Appareillage (50, 60) selon l'une des revendications 1 à 15, **caractérisé en ce que** ledit appareillage comprend un axe de pivotement, au moyen duquel l'adaptateur (60) peut être raccordé au dispositif (50) de manière à pouvoir pivoter.

17. Procédé destiné à la mesure des propriétés d'un oeil, en recourant à un appareillage (50, 60) selon l'une des revendications 1 à 16, selon lequel l'adaptateur (60) est positionné dans un trajet optique du dispositif (50) en vue de la mesure de l'au moins une topographie ou de l'au moins une courbure de l'oeil.

18. Procédé selon la revendication 17, **caractérisé par** les phases suivantes :
a. la mesure d'une première propriété de l'oeil en vue de l'obtention de premières valeurs de mesure ;
b. le pilotage, en particulier le positionnement, de l'adaptateur (60) en fonction des premières valeurs de mesure.

19. Procédé selon la revendication 18, **caractérisé en ce qu'**un espacement du dispositif (50) par rapport à l'oeil est déterminé au moyen du dispositif (50) et la position de l'adaptateur (60) est réglée et/ou surveillée à l'aide de cet espacement.
